# EUROPEAN PATENT APPLICATION

(11) **EP 2 431 068 A1**
(43) Date of publication of application: **21.03.2012**
(21) Application number: 11193369.3
(22) Date of filing: 07.03.2008
(51) Int. Cl.: A61M 25/10

(54) **A dilation catheter**

(62) Divisional of application: 08719317.3
(71) Applicant: London Equitable Limited in its capacity as Trustee of the Think Tank Trust, Mayfair London W1J 7SX (GB)
(72) Inventor: Zamboni, Paolo, 44100 Ferrara (IT)
(74) Representative: Freyria Fava, Cristina

(57) **Abstract**

A catheter (10) for treating stenotic sites in the human body (e.g. in the azygos vein) includes an expandable portion (12) having, when expanded, an arched shape. The arched shape may extends over an angle (α) of at least 90° degrees, and preferably of between 90° and 120° degrees, with a radius (R) of less than 3 centimetres, and preferably between 2 and 3 centimetres. The expandable portion (12) may include a flexible support member, and expandable members coupled to the support member to impart, when expanded, the desired arched shape to the flexible support member. Alternatively, the expandable portion (12) may includes an actuator member acting longitudinally of and sidewise to the expandable portion to impart thereto the desired arched shape.

## Description

### Field of the invention

This disclosure relates to dilation catheters.

This disclosure was developed by paying specific attention to its possible use in treating curved vessels such as, e.g. the azygos vein.

### Description of the related art

Dilation catheters are catheters provided with an expandable portion such as e.g. an inflatable "balloon" at its tip which is used during a catheterization procedure to enlarge a narrow opening or passage within the body. The unexpanded catheter (e.g. with the balloon deflated) is positioned, then inflated to perform the necessary procedure, and deflated again in order to be removed.

The art pertaining to expandable catheters is quite extensive, and a wide variety of technologies have been proposed for the production of such catheters.

A commonly accepted distinction in the art is between compliant and non-compliant catheters.

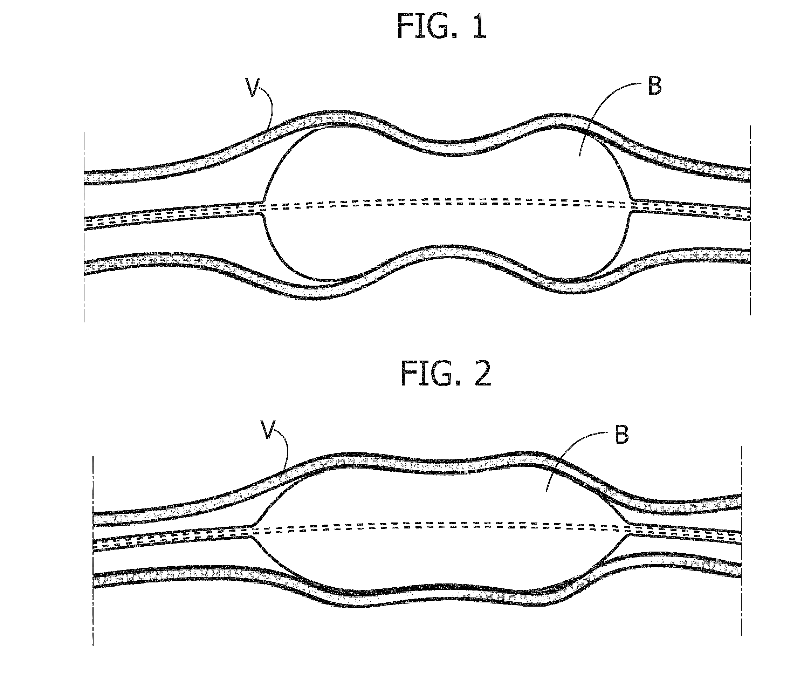

A compliant e.g. balloon catheter tends to hourglass around a stricture as schematically depicted in figure 1 annexed herewith. In that figure, reference V denotes a vessel being treated while B generally denotes the expandable portion of the catheter, e.g. the balloon located at the distal end of the catheter. Exemplary of compliant expandable catheters are documents such as WO-A-2006/124176 or WO-A-2006/062257.

By way of contrast, a non-compliant dilation catheter retains its shape as it generates force against the stricture such as schematically shown in figure 2. Exemplary of non-compliant expandable catheters are documents such as WO-A-2007/075256 or WO-A-2006/086516.

Irrespective of whether compliant or non-compliant, expandable catheters tend to extend rectilinearly when expanded. Also, a goal currently pursued in the design and manufacture of expandable catheters is to ensure that the expandable portion of the catheter expands as homogenously as possible around its periphery.

### Object and summary of the invention

Recent research in the area of multiple-sclerosis (MS) - see for instance a co-pending PCT application filed on February 26, 2008 by the same inventor - shows that a significant percentage of patients affected by MS exhibits steno-obstructive malformations affecting the azygos vein, frequently associated with similar pathology affecting either or both the internal jugular veins. Such malformations are in the form of septa, membranes, incomplete annulus structures formed in prenatal life, atresias.

The azygos vein ascends in the posterior mediastinum arching over the root of the right lung to transport deoxygenated blood from the posterior walls of the thorax and abdomen and from the thoraco-lumbar meningorachidian venous plexus into the superior vena cava. The vein is so named (azygous meaning "without twin" in ancient Greek) since it has no counterpart in the left side of the body.

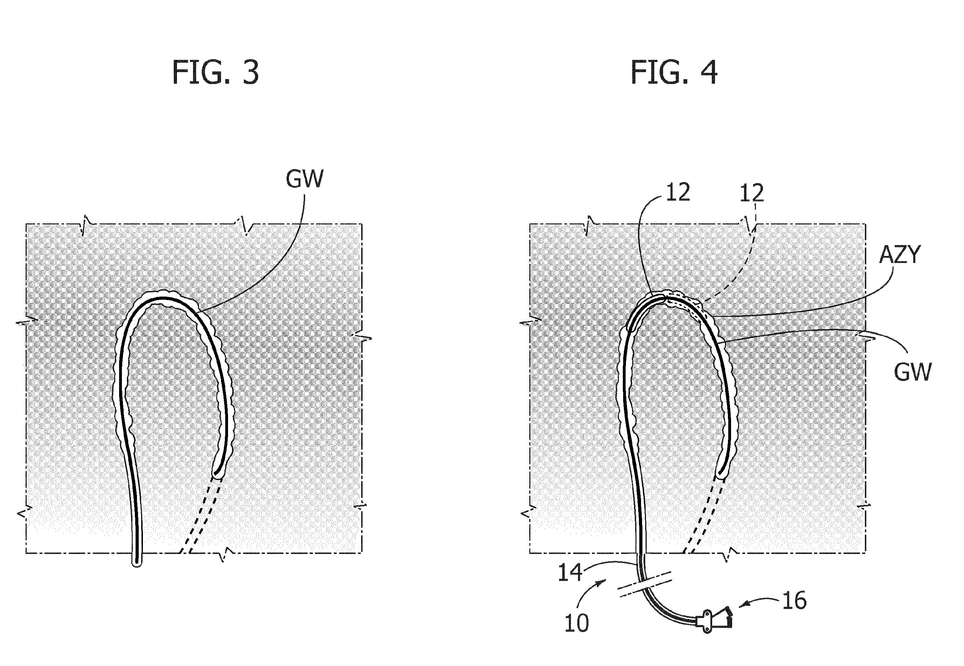

Figure 3 schematically shows, designated AZY, the arch of azygos vein (arcus venae azygou) which is an important anatomic feature of the human body. Typical values of the radius of curvature of the azygos vein are 2-3 centimetres.

Treating stenoses of the azygos vein by using conventional dilation catheters for can be hardly proposed. Using for that purpose a standard PTA (Percutaneous Transluminal Angioplasty) would in fact lead to the vein being forced into a rectilinear pattern during the PTA procedure. This would be a potential source of possible undesired effects such as anatomic dislocation, rupture, mediastinic haemorrhage, unless dilation is extremely reduced, with no appreciable effect in terms of desired stenosis treatment.

### Object and summary of the invention

The need is therefore felt for improved dilation catheter arrangements adapted for treating vessels having an arched, curvilinear path such as e.g. the azygos vein, especially when such arched/curvilinear path exhibits radiuses of a few centimetres (e.g. 2-3 centimetres) as is the case of the azygos vein in the vicinity of the superior vena cava.

The object of this disclosure is to provide such an improved catheter.

According to the invention, such an object is achieved by means of an expandable catheter having the features set forth in the claims that follow. The claims are an integral part of the disclosure of the invention provided herein.

In an embodiment, this disclosure provides a dedicated balloon catheter, exhibiting simultaneously a great degree of flexibility (i.e. steerability in reaching the expansion site) and optimal conformability to the shape of the vessel treated (e.g. the azygos vein arch).

In an embodiment, the catheter of this disclosure includes a distal balloon adapted to expand and to generate an appreciable dilation force while retaining an arched shape, i.e. a shape curved like an arch.

In an embodiment, the catheter of this disclosure exhibits a bellows-like or concertina-like structure on the side intended to form the outer (external) side of the arched pattern when expanded. When unexpanded, this structure can be easily folded over the catheter shaft as the catheter is led to the treatment site.

In certain embodiments, the catheter of the disclosure may include multiple chambers that communicate in a series arrangement (co-chambered arrangement), thus permitting the catheter to take on an arched pattern when expanded or inflated.

In an embodiment, the catheter of this disclosure includes the parallel of two co-chambered balloons arranged side-by side, namely an outer balloon and an inner balloon, wherein the inner balloon has a shorter length than the outer balloon. The inner balloon may thus constrain the longitudinal extension of the outer balloon thus bestowing an arched pattern to the outer balloon and the catheter as a whole.

### Brief description to the annexed representations

Exemplary embodiments of the invention will now be described, by way of example only, with reference to the annexed figures of drawing, wherein:
- figures 1 to 3 have already been discussed in the foregoing, and
- figure 4 schematically represents treating stenosis in an azygos vein; and
- figures 5 to 8 are representative of exemplary embodiments of an expandable catheter as described herein.

### Detailed description of exemplary embodiments

In the following description, numerous specific details are given to provide a thorough understanding of embodiments. The embodiments can be practiced without one or more of the specific details, or with other methods, components, materials, etc. In other instances, well known structures, materials or operations are not shown or described in detail to avoid obscuring aspects of the embodiments.

Reference throughout this specification to "one embodiment" or "an embodiment" means that a particular feature, structure or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures or characteristics may be combined in any suitable manner in one or more embodiments.

The headings provided herein are for convenience only and do not interpret the scope or meaning of the embodiments.

In figure 4, reference numeral 10 denotes as a whole an expandable catheter for use in treating (via Percutaneous Transluminal Angioplasty or PTA) stenosis of a vessel in a patient's body. The vessel has an arched or curvilinear path with a small radius of curvature (e.g. 2-3 centimetres). The azygos vein is exemplary of such a vessel.

In the embodiment shown, the catheter 10 includes an expandable portion ("balloon") 12 located at the distal end of an elongated flexible formation 14 (currently referred to as "shaft").

In a standard PTA procedure, the catheter 10 is introduced into a patient's body e.g. via the femoral vein and then advanced along a guide wire GW to locate the expandable portion 12 in correspondence with the stenotic site to be treated. Once located at the site to be treated, the expandable portion 12 is expanded by acting on expansion control means 16 located at the proximal end of the introducer member 14.

In an embodiment, the expandable portion 12 is a balloon structure which is expanded by inflation. The expansion means 16 may take the form of a pumping means adapted to convey along the introducer structure 14 fluid pressure to inflate the balloon 12 via pathways provided along the introducer member 14.

A PTA procedure may include one or more inflation/deflation cycles of the balloon 12. Once the procedure is completed, the balloon 12 may be finally deflated and the catheter 10 may be extracted from the patient's body by sliding it backwards along the guide wire GW, which is also final extracted from the patient's body.

Such a PTA procedure and the catheter features described so far are conventional in the art and do not require further detailed description herein.

Unless otherwise indicated in the following, this also refers to the technology involved in constructing and producing the catheter 10 and the component parts thereof.

Also, those of skill in the art will promptly appreciate that while a balloon or balloons will be primarily referred to herein as exemplary of expandable structure(s), the scope of this disclosure is in no way limited to such expandable structures.

In certain embodiments of this disclosure, such an expandable structure can include e.g. of mechanically expandable structures such as e.g. a slotted tube that radially expands when axially contracted. In an embodiment, axial contraction may be achieved under the action of flexible, axially retractable traction member adapted to be slid backwards along the guide wire GW.

Similarly, use of "memory shape" materials or superelastic materials (such as Nitinol, this material being well known for use in angioplasty devices) is within the scope of this disclosure.

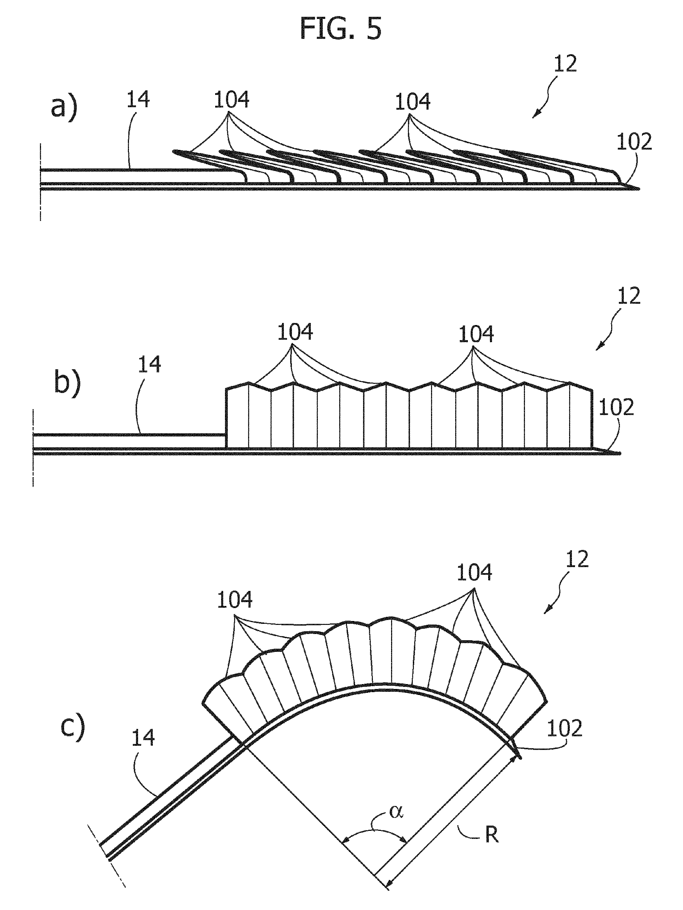

Figures 5 to 8 are representative of four exemplary embodiments of a catheter as disclosed herein.

Each of the figures 5 to 8 is comprised of three sections (designated "a", "b", and "c", respectively) which are representative of the condition of the expandable portion 12 of the catheter 10:
- when introduced into the patient's body and advanced towards the stenotic site to be treated (section "a");
- at an intermediate step of the expansion process at the site being treated (section "b"); and
- when fully expanded (section "c").

In the exemplary embodiments of figures 5 to 7, the distal tip 12 is comprised of a flexible support member 100 having a distal, "streamlined" end 102 adapted to facilitate advancement of the catheter towards the site to be treated.

The flexible member 100 may be a strip-like member of plastics material compatible for use within the human body. Materials currently used for the sheath introductory element 14 are exemplary of such materials. In certain embodiments, the flexible member 100 may be a wire-like member. Any other shapes (such as e.g. a helix shape) adapted to provide flexibility as required to negotiate the tortuous pathway to the implantation site and permit the tip portion 12 to take on an arched shape when expanded may be taken into account.

Coupled with the support member 100 are expandable (e.g. inflatable) formations that, when expanded, provide an expanded structure (see sections of figures 5 to 7 designated "c") according to a general arched path as better detailed in the following.

In the embodiment of figure 5, the formations in question are comprised of a plurality of chambers 104 of a flexible material (as currently used for manufacturing balloon catheters) connected - or "co-chambered" - in series in order to be simultaneously inflated by inflation fluid provided from the proximal end 16 of the insertion element 14 along pathways provided therein: as already indicated, providing such inflation fluid over the insertion element 14 is conventional in the art and does not require more detailed disclosure herein.

When the catheter 10 is introduced into the patient's body, the chambers 104 are not inflated and thus lie against the member 100 (possibly covered by a protection sheath to be axially withdrawn when the portion 1 reaches the treatment site).

As better detailed in section "b" of figure 5, the chambers 104 jointly comprise a bellows-like or concertina-like structure arranged on one side of the member 100, which is substantially inextensible. In addition to radial expansion of the catheter tip 12 as required for the PTA procedure, expansion of the inflatable chambers 104 also tends to provide longitudinal extension thereof which is constrained by the member 100.

As a result of such constriction or contraction action, when the chambers 104 are completely inflated (see section "c" of figure 5) the catheter tip 12 will assume an arched shape with a radius R extending over an angle α (alpha).

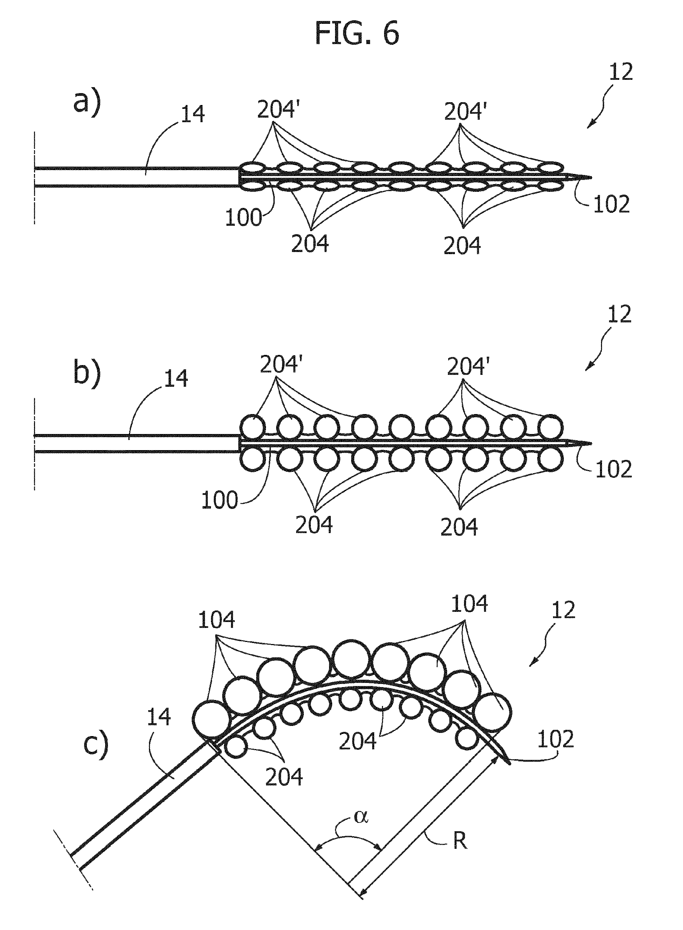

While in the embodiment of figure 4 the expandable members 104 are all locate on one side of the member 100, in the embodiment of figure 6, expandable members in the form of inflatable chambers or bubbles 204 and 204' expandable as result of the inflation fluid provided over the introductory element 14 are arranged on both sides of the member 100.

When the catheter 10 is introduced into the patient's body, the chambers 204, 204' are not inflated and thus lie against the member 100 (again, possibly covered by a protection sheath to be axially withdrawn when the portion 1 reaches the treatment site).

Inflation fluid provided along the introduction shaft 14 causes gradual inflation of the chambers 204, 204' (see section "b") of figures. The chambers 204 are however constructed in such a way that, when fully inflated (see section "c" of figure 6) they generally exhibit inflated radial sizes smaller than the corresponding inflated radial sizes of the chambers 204' located on the opposite side of the member 100.

As a result of the different inflated sizes of the bubbles 204, 204' on either sides of the member 100, the expanded tip will again assume an arched shape with a radius R and extending over an angle α (alpha).

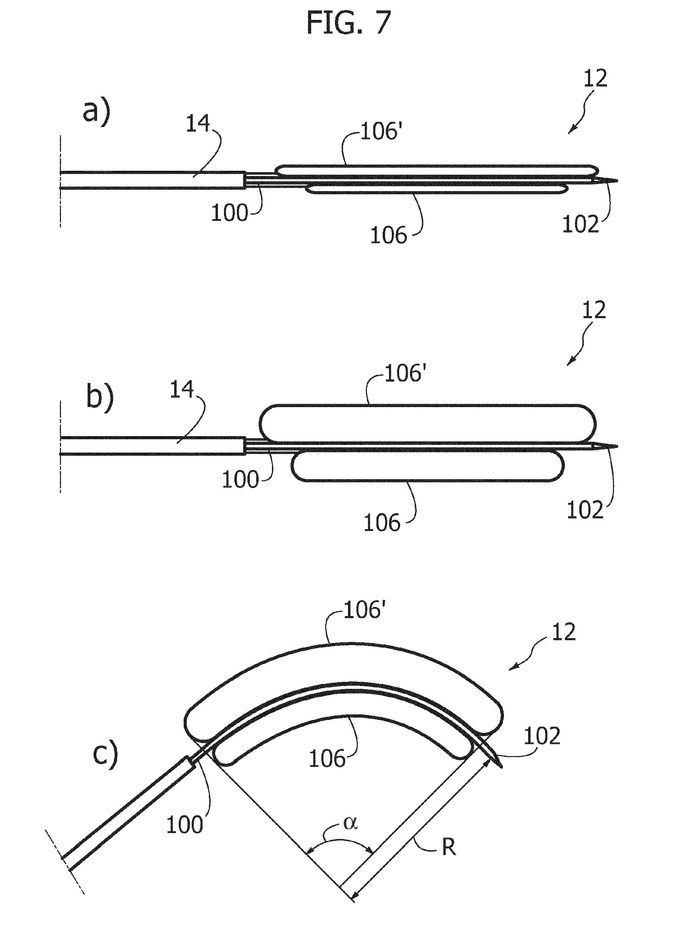

The embodiment of figure 7 is somewhat similar to the embodiment of figure 6, in that inflatable chambers are again provided in the form of one or more "inner" balloons 106 and one or more "outer" balloons 106'. The inner balloon or balloons 106 are configured in such a way to be axially shorter than the outer balloon or balloons 106'.

Consequently, while laying generally flat against the core member 100 when the catheter is introduced in the patient's body (see section "a" of figure 7), as inflation progresses (see section "b" and "c" of figure 7) the "shorter" inner balloon or balloons 106 will tend to axially constrain the expansion of the outer, "longer" balloon or balloons 106'. As a consequence of this constriction or contraction action, the expanded tip 12 of the catheter, when fully expanded, will again extend along an arched pattern with a radius R extending over an angle α (alpha).

The embodiments of Figures 5 to 7 are thus based on the concept of having an expandable portion 12 including a flexible support member 100, and expandable members (e.g. 104; 204, 204'; 106, 106') that are coupled to the support member 100 to impart, when expanded, the desired arched shape to the flexible support member 100 and to the expandable portion 12 as a whole.

In the embodiment of Figure 5, the expandable members 104 are all arranged on one side of the support member 100. In the embodiments of Figures 6 and 7, the expandable members 204, 204' and 106, 106' are arranged on both sides of the support member 100, with the expandable members (e.g. 204'; 106') located on the "inner" side of the support member 100 adapted to expand more than the expandable members 204; 106 located on the inner side of the support member 100.

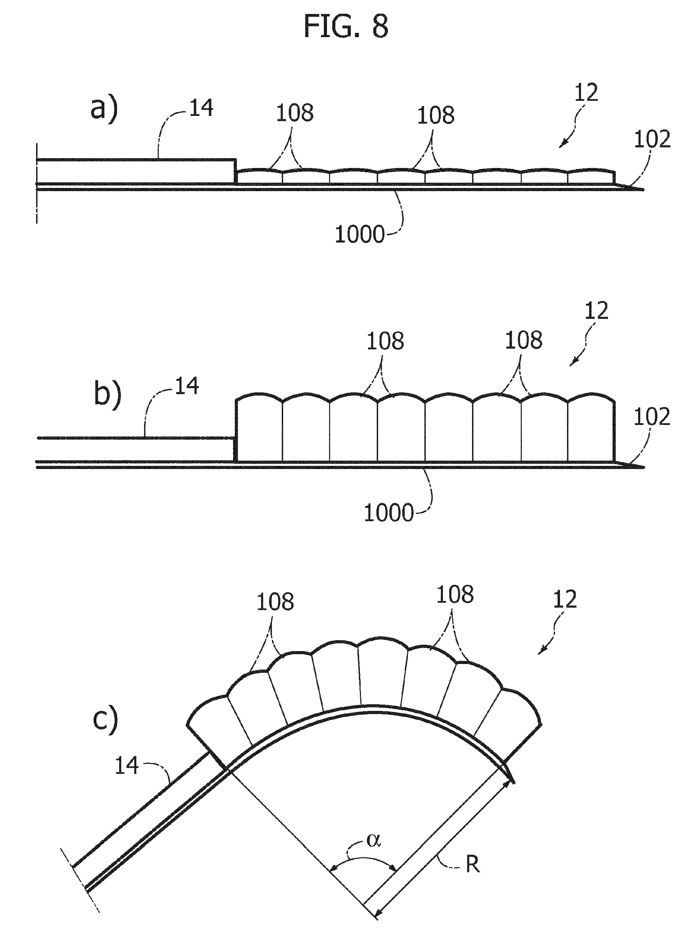

Conversely, in the embodiment of figure 8, the expandable portion 12 includes an actuator member, such as e.g. a flexible traction member 1000, acting longitudinally of and sidewise to the expandable portion 12 to impart thereto the desired arched shape.

In the exemplary embodiment illustrated, the expandable portion 12 again includes a plurality of "co-chambered" inflatable elements 108 adapted to receive inflation fluid from the introducer member 14 to ensure radial expansion of the catheter tip 12 as required for the PTA procedure.

Once inflated, the elements 108 are each of a roughly frustum-like or barrel shape and would tend to give rise to a sort of cylindrical worm-like structure overall (see figure 8, section "b").

The flexible traction member 1000 connects the elements 108 along one generatrix (i.e. sidewise) of the cylindrical structure. The traction member 1000 may be e.g. in the form a flexible metal wire adapted to be slidably retracted within the introducer member 14 as a result of being pulled from the proximal end thereof. Traction exerted via the member 1000 and applied sidewise to the expanded catheter tip 12 will again cause the expanded tip 12 to extend along an arched pattern with a radius R extending over an angle α (alpha).

A similar result may be obtained by using a flexible metal wire adapted to be slidably advanced within the introducer member 14 as a result of being pushed into the introducer 14 from the proximal end thereof. The forward thrust exerted via the member 1000 and applied sidewise to the expanded catheter tip 12 will again cause the expanded tip 12 to extend along an arched pattern with a radius R extending over an angle α (alpha).

Obviously, the directions of bending will be opposite depending on whether a pulling (i.e. traction) or pushing (i.e. thrust) member 100 is used.

One or more markers (such as e.g. radio-opaque markers) 12 will permit the practitioner to properly orientate the tip 12 in such a way that the arched pattern of the expanded tip will properly matches the arched pattern of the vessel being treated.

The schematic representation of figure 4 shows that PTA treatment of vessels such as the azygos vein AZY may take place in different steps.

For instance, by assuming e.g. that the arched pattern of the vessel to be treated extends over 180° (approximately) a catheter 10 as disclosed herein, wherein the expanded tip 12 extends over an angle α (alpha) of e.g. 90° degrees can be used to perform e.g. a two-step PTA procedure.

In a first step (full lines in Figure 4) , a first, proximal portion of the vessel over the first 90° degrees of its arched pattern is treated.

Once the PTA procedure is completed over that proximal portion of the vessel, the tip 12 can be radially contracted (e.g. deflated - non necessarily completely) and then advanced down the vessel to bring the tip 12 in correspondence with a further distal portion extending over further 90° degrees.

Once advanced, the tip 12 may be again expanded to perform a second step (broken lines in Figure 4) of PTA procedure over the distal portion of the vessel.

Obviously, the order (proximal-distal) of practising the various PTA procedures may be reversed (distal-proximal) if operational requirements so dictate. Also, more than two PTA (i.e. "ballooning") steps can be performed subsequently to cover the angular extension of the vessel to be treated. It will thus be appreciated that while an angular extension of α (alpha) of e.g. 90° degrees may be contemplated for an embodiment of this disclosure, different values, both smaller and larger, may be contemplated.

For instance, certain embodiments of this disclosure may provided for angular extensions (alpha) in the range of (at least) 90° - 120° degrees.

Embodiments of this disclosure may contemplate values in the range between 7 and 12 millimetres as the diameter of the tip 12 when expanded.

Embodiments of this disclosure may contemplate values in the range between 2 and 8 centimetres as the length of the tip 12 (as measured in the direction of the extension of the members 100 or 1000).

Embodiments of this disclosure provide for the expandable tip being comprised of one or more inflatable members adapted to bear inflation pressures up to 8 atmospheres.

Embodiments of this disclosure provide for values of the radius of curvature R of the tip 12 when inflated and curved in the range between 2 and 3 centimetres.

The expandable structure of the tip 12 may be of the compliant or semi-compliant type.

Embodiments of this disclosure provide for the introducer element 14 having a length in the range between 100 and 120 centimetres provided with a flexible, slidable and kink-resistant structure adapted to negotiate tortuous advancement path. These embodiments are fully compatible with using introducers of 6-7 French gauge.

All the quantitative data provided herein are to be understood by taking into account the inherent tolerances involved in realising and measuring the quantities indicated.

Also, without prejudice to the underlying principles of the invention, the details and embodiments may vary, also significantly, with respect to what has been described by way of example only, without departing from the scope of the invention as defined in the annexed claims.

## Claims

1. A catheter (10) having an expandable portion (12) for treating stenotic sites in body vessels, **characterised in that** said expandable portion (12) has, when expanded, an arched shape, wherein said expandable portion (12) includes:
- at least one inflatable member (108) and
- an actuator member (1000) acting longitudinally of and sidewise to said expandable portion (108) to impart said arched shape to said expandable portion (108).

2. The catheter of claim 1, wherein said arched shape extends over an angle (α) of at least 90° degrees, and preferably of between 90° and 120° degrees.

3. The catheter of either of claims 1 or 2, wherein said arched shape has a radius (R) of less than 3 centimetres, and preferably between 2 and 3 centimetres.

4. The catheter of any of the previous claims, wherein said expandable portion (12), when expanded, has a diameter between 7 and 12 millimetres.

5. The catheter of any of the previous claims, wherein said expandable portion (12), when expanded, has a length of between 2 and 8 centimetres.

6. The catheter of claim 1, wherein said at least one inflatable member (108) has an inflation pressure of up to 8 atmospheres.

7. The catheter of any of the previous claims, including an introducer member (14) having said expandable portion (12) at the distal end thereof, wherein said introducer member (14) has a length of between 100 and 120 centimetres.

8. The catheter of any of the previous claims, including at least one marker to properly orientate said expandable portion (12) to cause said arched shape to match the curvature of the site treated.

9. The catheter of any of the previous claims, wherein said expandable portion (12) includes a plurality of "co-chambered" inflatable elements (108).

10. The catheter of claim 9, wherein said plurality of inflatable elements (108) are each of a substantially frustum-like or barrel shape giving rise to a cylindrical worm-like structure.

11. The catheter of claim 10, wherein said flexible traction member (1000) connects said inflatable elements (108) along one generatrix of the cylindrical structure.

12. The catheter of any of claims 7 to 11, wherein said flexible traction member (1000) is in the form a flexible metal wire adapted to be slidably retracted within said introducer member (14) when pulled from the proximal end thereof.
